# EUROPEAN PATENT APPLICATION

(11) **EP 4 201 957 A1**
(43) Date of publication of application: **28.06.2023**
(21) Application number: 21858594.1
(22) Date of filing: 19.08.2021
(51) Int. Cl.: C07K 16/22, C12N 15/70

(54) **MODIFIED ANTIBODY AND METHOD FOR MANUFACTURING SAME**

(30) Priority: 19.08.2020 KR 20200103945
(71) Applicant: Pharmabcine Inc., Daejeon 34047 (KR)
(72) Inventor: KO, Jongil, Daejeon 34047 (KR); HA, Jung Min, Daejeon 34047 (KR); LEE, Joo Hyoung, Daejeon 34047 (KR); NAM, Ju Ryoung, Daejeon 34047 (KR); LEE, Weon Sup, Daejeon 34047 (KR)
(74) Representative: Garavelli, Paolo
(86) International application number: PCT/KR2021/010984
(87) International publication number: WO 2022/039507

(57) **Abstract**

The present invention relates to an improved modified antibody, of which solubility is increased and which can be concentrated to have a high concentration and a method for manufacturing same, and to a modified antibody in which amino acids located in the framework region of the antibody are substituted, and a method for manufacturing same.

## Description

### [Technical Field]

The present invention relates to an improved modified antibody having increased solubility and capable of being concentrated at a high level and a method for producing the same, and more specifically, the present invention relates to a modified antibody in which amino acids in the skeleton thereof are substituted and a method for producing the same.

### [Background Art]

Antibodies are used as therapeutic agents highly effective in the treatment of diseases such as cancer and autoimmune diseases. Antibody developability is evaluated in consideration of high yield, stable formulation, high physicochemical and *in vivo* stability, high solubility, and PK characteristics (Next generation antibody drugs: pursuit of the 'High-hanging fruit'. Nat. Rev. Drug Discovery., 2018, 17:197-223.)

Antibodies, protein pharmaceuticals, may be chemically unstable due to changes in covalent bonds or physically unstable due to deformation of the three-dimensional spatial structure. Antibodies may be chemically unstable due to hydrolysis, oxidation, deamidation, disulfide modification, or racemization, and may be physically unstable due to aggregation, adsorption, or dissolution.

The instability of the antibodies may affect solubility, efficacy, or the like and reduce the stability and solubility of the antibody, thus causing a decrease in productivity and production yield, as well as an increase in aggregation propensity, which increases the risk of immunogenicity.

Under this technical background, the present inventors have made efforts to develop a modified antibody that can be concentrated at a high level through improvement in the solubility of the antibody. As a result, the present inventors found that concentration was possible at a high level based on improved solubility through amino acid substitution at a specific position and completed the present invention based thereon.

### [Disclosure]

Therefore, it is one object of the present invention to provide an improved modified antibody having increased solubility and a fragment thereof.

It is another object of the present invention to provide a method of producing the modified antibody and fragment thereof.

In accordance with one aspect of the present invention, the above and other objects can be accomplished by the provision of a modified antibody and a fragment thereof in which at least one amino acid selected from the group consisting of amino acids at positions 13 and 42 of a heavy-chain variable region (based on the AHO numbering system) and an amino acid at the position 94 of a light-chain variable region (based on the AHO numbering system) is substituted.

In accordance with another aspect of the present invention, provided is a method of producing a modified antibody and a fragment thereof in which at least one amino acid selected from the group consisting of amino acids at positions 13 and 42 of a heavy-chain variable region (based on the AHO numbering system) and an amino acid at the position 94 of a light-chain variable region (based on the AHO numbering system) is substituted.

### [Description of Drawings]

FIG. 1 shows the results of antibody structure modeling and hot spot analysis.
FIG. 2 shows the results of expression test for L11T, V13S, and V103S (based on the AHO numbering system, hereinafter referred to as "L11T", "V12S", and "V93S") in antibody hotspots.
FIG. 3 shows the results of expression test for N42T, Q77R, and N94S (based on the AHO numbering system, hereinafter referred to as "N35T", "Q61R", and "N76S") in antibody hotspots.
FIG. 4 shows the results of expression test for V13S, N42T, and N94S (based on the AHO numbering system, hereinafter referred to as "V12S", "N35T", and "N76S") in antibody hotspots.
FIGS. 5A to 5G show results of antibody purification through SDS-PAGE.
FIG. 6 shows results of thermal stability analysis of the anti-Ang2 antibody according to the present invention.
FIGS. 7A to 7C show the results of evaluation of the anti-angiogenic efficacy of the anti-Ang2 antibody according to the present invention using a mouse CNV (choroidal neovascularization) model.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

The present inventors found that a modified antibody and a fragment thereof in which at least one amino acid selected from the group consisting of amino acids at positions 13 (V12) and 42 (N35) of the heavy-chain variable region (based on the AHO numbering system) and an amino acid at the position 94 (N76) of the light-chain variable region (based on the AHO numbering system) is substituted exhibit improved solubility, can be concentrated to a high level and thus exhibit improved productivity.

In particular, the present inventors found that a modified antibody and a fragment thereof in which the amino acid at position 13 (V12) in the heavy-chain variable region is substituted with serine (S), the amino acid at position 42 (N35) in the heavy-chain variable region is substituted with threonine (T), or the amino acid at position 94 (N76) is substituted with serine (S) in the light-chain variable region wherein the numbering is based on the AHO numbering system exhibit improved solubility, can be concentrated to a high level and thus exhibit improved productivity.

Based thereon, in one aspect, the present invention is directed to a modified antibody and a fragment thereof in which at least one amino acid selected from the group consisting of amino acids at positions 13 and 42 of the heavy-chain variable region (based on the AHO numbering system) and an amino acid at position 94 of the light-chain variable region (based on the AHO numbering system) is substituted.

In another aspect, the present invention is directed to a method for producing a modified antibody and a fragment thereof in which at least one amino acid selected from the group consisting of amino acids at positions 13 and 42 of the heavy-chain variable region (based on the AHO numbering system) and an amino acid at position 94 of the light-chain variable region (based on the AHO numbering system) is substituted.

The positions of amino acids for the antibodies or fragments thereof disclosed herein may be defined or identified based on the AHO numbering system. In some cases, the position of amino acid of the antibody or fragment thereof disclosed herein may mean the position sequentially numbered from No. 1 corresponding to the amino acid starting at the N-terminus of the amino acid sequences of the heavy-chain variable region of SEQ ID NO: 34 and the light-chain variable region of SEQ ID NO: 32.

In one embodiment, amino acids at position 13 and position 42 (based on the AHO numbering system) in the heavy-chain variable region and the amino acid at position 94 (based on the AHO numbering system) in the light-chain variable region may be substituted with serine (S) or threonine (T).

In specific embodiments, the substitutions of amino acids may include substitutions of amino acids at one or more positions (based on the AHO numbering system) selected from the group consisting of:
substitution of valine (V) at position 13 in the heavy-chain variable region with serine (S);
substitution of asparagine (N) at position 42 in the heavy-chain variable region with threonine (T); and
substitution of asparagine (N) at position 94 in the light-chain variable region with serine (S).

In specific embodiments of the present invention, the substitution of amino acids may include substitution of amino acids at the following positions (based on the AHO numbering system):
substitution of valine (V) at position 13 in the heavy-chain variable region with serine (S);
substitution of asparagine (N) at position 42 in the heavy-chain variable region with threonine (T);
substitution of asparagine (N) at position 94 in the light-chain variable region with serine (S);
substitution of valine (V) at position 13 in the heavy-chain variable region with serine (S), and substitution of asparagine (N) at position 42 of the heavy-chain variable region with threonine (T);
substitution of valine (V) at position 13 of the heavy-chain variable region with serine (S), and substitution of asparagine (N) at position 94 of the light-chain variable region with serine (S);
substitution of asparagine (N) at position 42 of the heavy-chain variable region with threonine (T), and substitution of asparagine (N) at position 94 of the light-chain variable region with serine (S); or
substitution of valine (V) at position 13 of the heavy-chain variable region with serine (S), substitution of asparagine (N) at position 42 of the heavy-chain variable region with threonine (T), and substitution of asparagine (N) at position 94 of the light-chain variable region with serine (S).

The substitutions of amino acids described above lead to improvement in the solubility of the antibody, concentration to high levels and improvement in productivity.

Unlike the above amino acid substitution, substitution of leucine at position 11 in the heavy-chain variable region (based on the AHO numbering system) with threonine, substitution of valine at position 103 in the heavy-chain variable region (based on the AHO numbering system) with serine, or substitution of glutamine at position 77 in the light-chain variable region (based on the AHO numbering system) with arginine was not found to exhibit the desired effect of improving solubility.

In specific embodiments, the modified antibody or fragment thereof according to the present invention may include one or more framework regions selected from the group consisting of:
QVQLVESGGGLX₁KPGGSLRLSCAAS (in which X₁ is S);
MX₂WVRQAPGKGLEWVSS (in which X₂ is T) ; and
NLQSGVSSQFSGSGSGTDFTLTIX₃SLQPEDSATYYC (in which X₃ is S) .

Specifically, the anti-Ang2 antibody clone, 04, including the heavy-chain variable region of SEQ ID NO: 34 and the light-chain variable region of SEQ ID NO: 32 may include substitutions with serine (S) or threonine (T) of one or more amino acids selected from the group consisting of amino acids at positions 12 and 35 in the heavy-chain variable region and amino acid at position 76 in the light-chain variable region.

In one embodiment, the anti-Ang2 antibody clone, 04, including the heavy-chain variable region of SEQ ID NO: 34 and the light-chain variable region of SEQ ID NO: 32 may include one or more amino acid substitutions selected from the group consisting of:
substitution of valine (V) at position 12 in the heavy-chain variable region with serine (S);
substitution of asparagine (N) at position 35 in the heavy-chain variable region with threonine (T); and
substitution of asparagine (N) at position 76 in the light-chain variable region with serine (S).

Specifically, according to the present invention, the anti-Ang2 antibody clone, O4, may include the following amino acid substitution:
substitution of valine (V) at position 12 in the heavy-chain variable region with serine (S);
substitution of asparagine (N) at position 35 in the heavy-chain variable region with threonine (T);
substitution of asparagine (N) at position 76 in the light-chain variable region with serine (S);
substitution of valine (V) at position 12 of the heavy-chain variable region with serine (S), and substitution of asparagine (N) at position 35 of the heavy-chain variable region with threonine (T);
substitution of valine (V) at position 12 of the heavy-chain variable region with serine (S), and substitution of asparagine (N) at position 76 of the light-chain variable region with serine (S);
substitution of asparagine (N) at position 35 in the heavy-chain variable region with threonine (T) and substitution of asparagine (N) at position 76 in the light-chain variable region with serine (S); or
substitution of valine (V) at position 12 of the heavy-chain variable region with serine (S), substitution of asparagine (N) at position 35 of the heavy-chain variable region with threonine (T), and substitution of asparagine (N) at position 76 of the light-chain variable region with serine (S).

Attempts to improve the physical properties of O4, an anti-Ang2 antibody clone including the heavy-chain variable region of SEQ ID NO: 34 and the light-chain variable region of SEQ ID NO: 32 were made. The results showed that when O4 has a framework region having a sequence of QVQLVESGGGLX₁KPGGSLRLSCAAS (wherein X₁ is S) in FR1 of the heavy-chain variable region, or a framework region having a sequence of MX₂WVRQAPGKGLEWVSS (wherein X₂ is T) in FR3, or QVQLVESGGGLX₁KPGGSLRLSCAAS (wherein X₁ in FR1 is S) in FR1 and MX₂WVRQAPGKGLEWVSS in FR3 (wherein X₂ is T), it can be concentrated at a high level due to the improved solubility of the antibody and thus exhibit improved productivity.

The results showed that, when O4 has a framework region having a sequence of NLQSGVSSQFSGSGSGTDFTLTIX₃SLQPEDSATYYC (wherein X₃ is S) in FR3 of the light-chain variable region, it can be concentrated at a high level due to the improved solubility of the antibody and thus exhibit improved productivity.

The results showed that, when O4 has a framework region having a sequence of QVQLVESGGGLX1KPGGSLRLSCAAS (wherein X₁ is S) in FR1 of the heavy-chain variable region and a framework region having a sequence of NLQSGVSSQFSGSGSGTDFTLTIX₃SLQPEDSATYYC (wherein X₃ is S) in FR3 of the light-chain variable region, or
O4 has a framework region having a sequence of MX₂WVRQAPGKGLEWVSS (wherein X₂ is T) in FR3 of the heavy-chain variable region and a framework region having a sequence of NLQSGVSSQFSGSGSGTDFTLTIX₃SLQPEDSATYYC (wherein X₃ is S) in FR3 of the light-chain variable region, or
O4 has a framework region having a sequence of QVQLVESGGGLX1KPGGSLRLSCAAS (wherein X₁ is S) in FR1 of the heavy-chain variable region, a framework region having a sequence of MX₂WVRQAPGKGLEWVSS (wherein X₂ is T) in FR3 of the heavy-chain variable region, and a framework region having a sequence of NLQSGVSSQFSGSGSGTDFTLTIX₃SLQPEDSATYYC (wherein X₃ is S) in FR3 of the light-chain variable region, it can be concentrated at a high level due to the improved solubility of the antibody and thus exhibit improved productivity.

In some cases, the modified antibody or fragment thereof according to the present invention may be an antibody or antigen-binding fragment thereof that binds to Ang2 (angiopoietin-2).

In this case, the antibody or antigen-binding fragment thereof may include the following:
a heavy-chain variable region including a heavy-chain CDR1 selected from the group consisting of SEQ ID NOS: 1, 7, 13, 19 and 25,
a heavy-chain CDR2 selected from the group consisting of SEQ ID NOS: 2, 8, 14, 20 and 26, and
a heavy-chain CDR3 selected from the group consisting of SEQ ID NOS: 3, 9, 15, 21, 27, 43, 44 and 45, and
a light-chain variable region including a light-chain CDR1 selected from the group consisting of SEQ ID NOS: 4, 10, 16, 22, and 28,
a light-chain CDR2 selected from the group consisting of SEQ ID NOS: 5, 11, 17, 23 and 29, and
a light-chain CDR3 selected from the group consisting of SEQ ID NOS: 6, 12, 18, 24 and 30.

| Sequence name | | Amino acid sequence | SEQ. ID No. |
|---|---|---|---|
| 3 | Heavy-chain CDR1 | GFSFDDYA | 1 |
| | Heavy-chain CDR2 | IKDDGSQT | 2 |
| | Heavy-chain CDR3 | TTEGLMNGLHFDM | 3 |
| | Light-chain CDR1 | SSNIGAGYD | 4 |
| | Light-chain CDR2 | GNN | 5 |
| | Light-chain CDR3 | QSYDSRLGVV | 6 |
| 4 | Heavy-chain CDR1 | GYSFTSYW | 7 |
| | Heavy-chain CDR2 | IYPGNSDT | 8 |
| | Heavy-chain CDR3 | TTEGLMNGLHFDM | 9 |
| | Light-chain CDR1 | QSLLHSLGDNY | 10 |
| | Light-chain CDR2 | LGS | 11 |
| | Light-chain CDR3 | MQSLQTPPYT | 12 |
| 8 | Heavy-chain CDR1 | GFTFSSYS | 13 |
| | Heavy-chain CDR2 | ISASDGAT | 14 |
| | Heavy-chain CDR3 | AKILAGYSGPMGGMDV | 15 |
| | Light-chain CDR1 | RDISNY | 16 |
| | Light-chain CDR2 | GAS | 17 |
| | Light-chain CDR3 | QQYYSYPLT | 18 |
| 41 | Heavy-chain CDR1 | GFAFGRYE | 19 |
| | Heavy-chain CDR2 | IDTGGGAK | 20 |
| | Heavy-chain CDR3 | TTEGLMNGLHFDM | 21 |
| | Light-chain CDR1 | QAISTW | 22 |
| | Light-chain CDR2 | TAS | 23 |
| | Light-chain CDR3 | QQLNSYPYT | 24 |
| 46 | Heavy-chain CDR1 | GFTFDDCA | 25 |
| | Heavy-chain CDR2 | ISGNSKNV | 26 |
| | Heavy-chain CDR3 | ARDPAYSQFDY | 27 |
| | Light-chain CDR1 | SSNVGGYP | 28 |
| | Light-chain CDR2 | TDY | 29 |
| | Light-chain CDR3 | ATWDDNLNGYV | 30 |
| O4 | Heavy-chain CDR3 | AKTLAGYSGPMGGMDV | 43 |
| O10 | Heavy-chain CDR3 | AKILVGYSGPMGGMDV | 44 |
| O12 | Heavy-chain CDR3 | AKSLASYSGPMGGMDV | 45 |

As used herein, the term "antibody" refers to an anti-Ang2 antibody that specifically binds to Ang2. A complete antibody and antigen-binding fragment of the antibody molecules falls within the scope of the present invention.

The whole antibody has a structure having two full-length light-chains and two full-length heavy-chains, and each light-chain is bonded to the heavy-chain by a disulfide bond. The heavy-chain constant region has gamma (γ), mu (µ), alpha (α), delta (δ) and epsilon (ε) types, and is subclassified into gamma 1 (γ1), gamma 2 (γ2), gamma 3 (γ3), gamma 4 (γ4), alpha 1 (α1), and alpha 2 (α2). The light-chain constant region has kappa (κ) and lambda (λ) types.

The antigen-binding fragment of an antibody or antibody fragment is a fragment that has antigen-binding capacity and includes Fab, F(ab'), F(ab')2, Fv and the like. Among the antibody fragments, Fab refers to a structure including a variable region of each of the heavy-chain and the light-chain, the constant region of the light-chain, and the first constant domain (CH1) of the heavy-chain, each having one antigen-binding site. Fab' is different from Fab in that it further includes a hinge region including at least one cysteine residue at the C-terminus of the CH1 domain of the heavy-chain. F(ab')2 is created by a disulfide bond between cysteine residues in the hinge region of Fab'. Fv is the minimal antibody fragment having only a heavy-chain variable region and a light-chain variable region. Two-chain Fv is a fragment wherein the variable region of the heavy-chain and the variable region of the light-chain are linked by a non-covalent bond, and single-chain Fv (scFv) is a fragment wherein the variable region of the heavy-chain and the variable region of the light-chain are generally linked by a covalent bond via a peptide linker therebetween, or are directly linked at the C-terminus, forming a dimer-shaped structure, like the two-chain Fv. Such antibody fragments may be obtained using proteases (e.g., Fab can be obtained by restriction-cleaving the complete antibody with papain, and the F(ab')2 fragment can be obtained by cleaving the complete antibody with pepsin), and may be also prepared using genetic recombination techniques.

In one embodiment, the antibody of the present invention is in an Fv form (for example, scFv) or a complete antibody form. In addition, the heavy-chain constant region may be selected from gamma (γ), mu (u), alpha (α), delta (δ) and epsilon (c) isotypes. For example, the constant region may be gamma 1 (IgG1), gamma 3 (IgG3) or gamma 4 (IgG4). The light-chain constant region may be kappa or lambda.

As used herein, the term "heavy-chain" encompasses both a full-length heavy-chain, which includes a variable domain (VH), containing an amino acid sequence having a variable region sequence sufficient for imparting specificity to an antigen and three constant domains (CH1, CH2 and CH3), and a fragment thereof. As used herein, the term "light-chain" encompasses both a full-length light-chain, which includes a variable domain (VL) containing an amino acid sequence having a variable region sequence sufficient for imparting specificity to an antigen and a constant domain (CL), and a fragment thereof.

The antibody of the present invention includes, but is not limited to, monoclonal antibodies, multispecific antibodies, human antibodies, humanized antibodies, chimeric antibodies, single chain FVs (scFVs), single-chain antibodies, Fab fragments, F(ab') fragments, disulfide-bond Fvs (sdFVs), anti-idiotypic (anti-Id) antibodies, epitope-binding fragments of such antibodies, and the like.

The term "monoclonal antibody" refers to an identical antibody, which is obtained from a population of substantially homogeneous antibodies, that is, each antibody constituting the population, excluding possible naturally occurring mutations that may be present in trivial amounts. Monoclonal antibodies are highly specific and are thus induced against a single antigenic site. Unlike conventional (polyclonal) antibody preparations that typically include different antibodies directed against different determinants (epitopes), each monoclonal antibody is directed against a single determinant on the antigen.

The term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The non-human (*e*.*g*., murine) antibody of the "humanized" form is a chimeric antibody including one or more amino acid sequences (e.g., CDR sequences) derived from one or more non-human antibodies (donor or source antibodies) containing minimal sequences derived from non-human immunoglobulins. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

As used herein, the term "human antibody" refers to a molecule derived from human immunoglobulin, in which all of the amino acid sequences constituting the antibody including a complementarity-determining region and a structural region are composed of human immunoglobulins.

A part of the heavy-chain and/or light-chain is identical to or homologous with the corresponding sequence in an antibody derived from a particular species or belonging to a particular antibody class or subclass, while the remaining chain(s) include "chimeric" antibodies (immunoglobulins) which are identical to or homologous with corresponding sequences in an antibody derived from another species or belonging to another antibody class or subclass, as well as fragments of such antibody exhibiting the desired biological activity.

As used herein, the term "antibody variable domain" refers to the light- and heavy-chain regions of an antibody molecule including the amino acid sequences of a complementarity-determining region (CDR; i.e., CDR1, CDR2, and CDR3) and a framework region (FR). VH refers to a variable domain of the heavy-chain. VL refers to a variable domain of the light-chain.

The term "complementarity-determining region" (CDR, that is, CDR1, CDR2, and CDR3), refers to an amino acid residue of the antibody variable domain, which is necessary for antigen binding. Each variable domain typically has three CDR regions, identified as CDR1, CDR2, and CDR3. The complementarity-determining region of the present invention includes a heavy-chain variable region including the heavy-chain CDR3 of SEQ ID NO: 1 and a light-chain variable region including the light-chain CDR3 of SEQ ID NO: 2.

Specifically, the antibody or an antigen-binding fragment thereof binding to Ang2 includes: a heavy-chain variable region including a heavy-chain CDR1 of SEQ ID NO: 1, a heavy-chain CDR2 of SEQ ID NO: 2 and a heavy-chain CDR3 of SEQ ID NO: 3, and a light-chain variable region including a light-chain CDR1 of SEQ ID NO: 4, a light-chain CDR2 of SEQ ID NO: 5 and a light-chain CDR3 of SEQ ID NO: 6,
a heavy-chain variable region including a heavy-chain CDR1 of SEQ ID NO: 7, a heavy-chain CDR2 of SEQ ID NO: 8 and a heavy-chain CDR3 of SEQ ID NO: 9, and a light-chain variable region including a light-chain CDR1 of SEQ ID NO: 10, a light-chain CDR2 of SEQ ID NO: 11 and a light-chain CDR3 of SEQ ID NO: 12,
a heavy-chain variable region including a heavy-chain CDR1 of SEQ ID NO: 13, a heavy-chain CDR2 of SEQ ID NO: 14 and a heavy-chain CDR3 of SEQ ID NO: 15, and a light-chain variable region including a light-chain CDR1 of SEQ ID NO: 16, a light-chain CDR2 of SEQ ID NO: 17 and a light-chain CDR3 of SEQ ID NO: 18,
a heavy-chain variable region including a heavy-chain CDR1 of SEQ ID NO: 19, a heavy-chain CDR2 of SEQ ID NO: 20 and a heavy-chain CDR3 of SEQ ID NO: 21, and a light-chain variable region including a light-chain CDR1 of SEQ ID NO: 22, a light-chain CDR2 of SEQ ID NO: 23 and a light-chain CDR3 of SEQ ID NO: 24,
a heavy-chain variable region including a heavy-chain CDR1 of SEQ ID NO: 25, a heavy-chain CDR2 of SEQ ID NO: 26 and a heavy-chain CDR3 of SEQ ID NO: 27, and a light-chain variable region including a light-chain CDR1 of SEQ ID NO: 28, a light-chain CDR2 of SEQ ID NO: 29 and a light-chain CDR3 of SEQ ID NO: 30,
a heavy-chain variable region including a heavy-chain CDR1 of SEQ ID NO: 13, a heavy-chain CDR2 of SEQ ID NO: 14 and a heavy-chain CDR3 of SEQ ID NO: 43, and a light-chain variable region including a light-chain CDR1 of SEQ ID NO: 16, a light-chain CDR2 of SEQ ID NO: 17 and a light-chain CDR3 of SEQ ID NO: 18,
a heavy-chain variable region including a heavy-chain CDR1 of SEQ ID NO: 13, a heavy-chain CDR2 of SEQ ID NO: 14 and a heavy-chain CDR3 of SEQ ID NO: 44, and a light-chain variable region including a light-chain CDR1 of SEQ ID NO: 16, a light-chain CDR2 of SEQ ID NO: 17 and a light-chain CDR3 of SEQ ID NO: 18, or
a heavy-chain variable region including a heavy-chain CDR1 of SEQ ID NO: 13, a heavy-chain CDR2 of SEQ ID NO: 14 and a heavy-chain CDR3 of SEQ ID NO: 45, and a light-chain variable region including a light-chain CDR1 of SEQ ID NO: 16, a light-chain CDR2 of SEQ ID NO: 17 and a light-chain CDR3 of SEQ ID NO: 18.

The term "framework region" (FR) refers to a variable domain residue other than a CDR residue. Each variable domain typically has four FRs, identified as FR1, FR2, FR3, and FR4. The inventors of the present application induced mutations in the framework regions with the goal of improving productivity and solubility in order to develop improved-productivity and high-concentration formulations.

The "Fv" fragment is an antibody fragment containing complete antibody recognition and binding sites. Such a region includes a dimer that consists of one heavy-chain variable domain and one light-chain variable domain substantially tightly covalently linked to each other, for example, through scFv.

A "Fab" fragment contains a variable domain and a constant domain of the light-chain and a variable domain and a first constant domain (CH1) of the heavy-chain. A F(ab')₂ antibody fragment generally includes a pair of Fab fragments covalently linked near the carboxyl terminal thereof via a hinge cysteine therebetween.

The "single-chain Fv" or "scFv" antibody fragment includes VH and VL domains of the antibody, wherein these domains are present in a single polypeptide chain. The Fv polypeptide may further include a polypeptide linker between the VH domain and the VL domain in order for the scFv to form a desired structure for antigen binding.

Functionally, the binding affinity of the antibody according to the present invention ranges from 10⁻⁵ M to 10⁻¹² M. The amino acid substitution according to the present invention may neither reduce the affinity for the antigen nor adversely affect the affinity.

For example, the binding affinity of the modified antibody according to the present invention to the antigen is 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M or 10⁻⁵ M to 10⁻⁶ M.

The modified antibody according to the present invention may include a heavy-chain variable region selected from the group consisting of SEQ ID NOS: 31, 33, and 36 and/or a light-chain variable region of SEQ ID NO: 35.

In a specific embodiment according to the present invention, the modified antibody according to the present invention may include the heavy-chain variable region of SEQ ID NO: 31 and the light-chain variable region of SEQ ID NO: 32;
a heavy-chain variable region of SEQ ID NO: 33 and a light-chain variable region of SEQ ID NO: 32;
a heavy-chain variable region of SEQ ID NO: 34 and a light-chain variable region of SEQ ID NO: 35;
a heavy-chain variable region of SEQ ID NO: 36 and a light-chain variable region of SEQ ID NO: 32;
a heavy-chain variable region of SEQ ID NO: 31 and a light-chain variable region of SEQ ID NO: 35;
a heavy-chain variable region of SEQ ID NO: 33 and a light-chain variable region of SEQ ID NO: 35; or
a heavy-chain variable region of SEQ ID NO: 36 and a light-chain variable region of SEQ ID NO: 35.

The modified antibody or fragment thereof according to the present invention may include not only sequences of antibodies but also biological equivalents thereto, as long as it can maintain the desired functions. For example, additional variations can be made to the amino acid sequence of the antibody in order to further improve the binding affinity and/or other biological properties of the antibody. Such variations include, for example, deletion, insertion and/or substitution of the amino acid sequence residues of the antibody. Such amino acid mutations are based on the relative similarity of amino-acid side-chain substituents, such as the hydrophobicity, hydrophilicity, charge and size thereof. It can be seen through analysis of the size, shape and type of amino-acid side-chain substituents that all of arginine, lysine and histidine are positively charged residues; alanine, glycine and serine have similar sizes; and phenylalanine, tryptophan and tyrosine have similar shapes. Thus, based on these considerations, arginine, lysine and histidine; alanine, glycine and serine; and phenylalanine, tryptophan and tyrosine are considered to be biologically functional equivalents.

When taking into consideration mutations having biologically equivalent activity, the antibody or a nucleic acid molecule encoding the same according to the present invention is interpreted to include a sequence having substantial identity with the sequence set forth in the sequence number. The term "substantial identity" means that a sequence has a homology of at least 90%, preferably a homology of at least 90%, most preferably at least 95%, at least 96%, at least 97%, at least 98%, and at least 99%, when aligning the sequence of the present invention and any other sequence so as to correspond to each other as much as possible and analyzing the aligned sequence using algorithms commonly used in the art. Alignment methods for sequence comparison are well-known in the art. The NCBI basic local alignment search tool (BLAST) is accessible through NCBI or the like, and can be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program can be found at www.ncbi.nlm.nih.gov/BLAST/blast help.html.

Based on this, the antibody or antigen-binding fragment thereof according to the present invention can have a homology of 90%, 91%, 92%, 93%, 94%, 95%, 96%, 97%, 98%, 99%, or more compared to the sequence disclosed herein or the entirety thereof. Homology can be determined through sequence comparison and/or alignment by methods known in the art. For example, the percentage sequence homology of the nucleic acid or protein according to the present invention can be determined using a sequence comparison algorithm (*i*.*e*., BLAST or BLAST 2.0), manual alignment, or visual inspection.

In another aspect, the present invention is directed to a nucleic acid encoding the modified antibody or fragment thereof.

The modified antibody or fragment thereof can be produced in a recombinant manner by isolating the nucleic acid encoding the antibody or antigen-binding fragment thereof of the present invention. The nucleic acid is isolated and inserted into a replicable vector, followed by further cloning (amplification of DNA) or further expression. Based thereon, in another aspect, the present invention is directed to a vector including the nucleic acid.

The term "nucleic acid" is intended to encompass both DNA (gDNA and cDNA) and RNA molecules, and a nucleotide, which is the basic constituent unit of nucleic acids, includes naturally derived nucleotides as well as analogues thereof, in which sugar or base moieties are modified. The sequence of the nucleic acid encoding heavy- and light-chain variable regions of the present invention can vary. Such variation includes addition, deletion, or non-conservative or conservative substitution of nucleotides.

In a specific embodiment according to the present invention, the nucleic acid may include the following sequence:
a sequence encoding a heavy-chain variable region of SEQ ID NO: 37 and a sequence encoding a light-chain variable region of SEQ ID NO: 38;
a sequence encoding a heavy-chain variable region of SEQ ID NO: 39 and a sequence encoding a light-chain variable region of SEQ ID NO: 38;
a sequence encoding a heavy-chain variable region of SEQ ID NO: 40 and a sequence encoding a light-chain variable region of SEQ ID NO: 41;
a sequence encoding a heavy-chain variable region of SEQ ID NO: 42 and a sequence encoding a light-chain variable region of SEQ ID NO: 38;
a sequence encoding a heavy-chain variable region of SEQ ID NO: 37 and a sequence encoding a light-chain variable region of SEQ ID NO: 41;
a sequence encoding a heavy-chain variable region of SEQ ID NO: 39 and a sequence encoding a light-chain variable region of SEQ ID NO: 41; or
a sequence encoding a heavy-chain variable region of SEQ ID NO: 42 and a sequence encoding a light-chain variable region of SEQ ID NO: 41.

The DNA encoding the antibody can be easily separated or synthesized using conventional procedures (for example, using an oligonucleotide probe capable of specifically binding to DNA encoding heavy and light-chains of the antibody). A variety of vectors are obtainable. Vector components generally include, but are not limited to, one or more of the following components: signal sequences, replication origins, one or more marker genes, enhancer elements, promoters, and transcription termination sequences.

As used herein, the term "vector" refers to a means for expressing target genes in host cells, and includes plasmid vectors, cosmid vectors, and viral vectors such as bacteriophage vectors, adenovirus vectors, retroviral vectors, and adeno-associated viral vectors. The nucleic acid encoding the antibody in the vector is operably linked to a promoter.

The term "operably linked" means functional linkage between a nucleic acid expression regulation sequence (e.g., an array of the binding site of the promoter, signal sequence, or transcription regulator) and another nucleic acid sequence, and enables the regulation sequence to regulate transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of a mammalian cell (e.g., a metallothionein promoter, a β-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

Optionally, the vector may be fused with another sequence in order to facilitate purification of the antibody expressed thereby. The sequence to be fused therewith may include, for example, glutathione S-transferase (Pharmacia, USA), maltose-binding protein (NEB, USA), FLAG (IBI, USA), 6x His (hexahistidine; Qiagen, USA) and the like.

The vector includes antibiotic resistance genes commonly used in the art as selectable markers, and examples thereof include genes conferring resistance to ampicillin, gentamycin, carbenicillin, chloramphenicol, streptomycin, kanamycin, geneticin, neomycin, and tetracycline.

In another aspect, the present invention is directed to a cell transformed with the above-mentioned vector. The cell used to produce the antibody of the present invention may be a prokaryote, yeast, or higher eukaryotic cell, but is not limited thereto.

Prokaryotic host cells such as *Escherichia coli,* strains of the genus *Bacillus,* such as *Bacillus subtilis* and *Bacillus thuringiensis, Streptomyces* spp., *Pseudomonas* spp. (for example, *Pseudomonas putida*)*, Proteus mirabilis* and *Staphylococcus* spp. (for example, *Staphylococcus carnosus*) may be used.

Interest in animal cells is the greatest, and examples of useful host cell lines include, but are not limited to, COS-7, BHK, CHO, CHOK1, DXB-11, DG-44, CHO/- DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing a modified antibody or fragment thereof in which at least one amino acid at position 12 or 35 of the heavy-chain variable region (based on the AHO numbering system) and/or an amino acid at position 76 of the light-chain variable region (based on the AHO numbering system) is substituted with serine (S) or threonine (T).

A nucleic acid encoding the modified antibody or fragment thereof may be introduced into cells. The cells may be cultured in various media. Any commercially available medium can be used as a culture medium without limitation. All other essential supplements well-known to those skilled in the art may be included in appropriate concentrations. Culture conditions such as temperature and pH are those that are conventionally used with host cells selected for expression, as will be apparent to those skilled in the art.

The recovery of the antibody or fragment thereof can be carried out, for example, by centrifugation or ultrafiltration to remove impurities from and purify the resulting product using, for example, affinity chromatography. Other additional purification techniques, such as anion or cation exchange chromatography, hydrophobic interaction chromatography, and hydroxyapatite (HA) chromatography, may be used.

In another aspect, the present invention is directed to a composition for preventing or treating tumors containing the antibody or fragment thereof as an active ingredient. The antibody may be an IgG or a fragment including a variable region, that is, ScFv or Fab. In addition, the variable region of the heavy-chain may be IgG1, IgG2, IgG3, or IgG4.

In another aspect, the present invention is directed to a pharmaceutical composition for preventing or treating eye diseases including (a) a pharmaceutically effective amount of the antibody or fragment thereof according to the present invention, and (b) a pharmaceutically acceptable carrier. In another aspect, the present invention is directed to a method for preventing or treating eye diseases including administering the antibody or fragment thereof according to the present invention to an eye disease patient. Furthermore, the present invention is directed to the use of the antibody or antigen-binding fragment thereof for inhibiting the mechanism of Ang2 and the use thereof for preventing or treating eye diseases based thereon.

Regarding eye diseases, the cornea is avascular tissue and must always maintain transparency to preserve vision. However, angiogenesis is also known to occur in the eye which causes ocular angiogenesis-related diseases. In other words, neovascularization in the cornea impairs the transparency of the eyeball, resulting in loss of vision, and neovascularization in the retina induces the formation of abnormal blood vessels and exudation of blood, causing blindness through degeneration of retinal cells.

Based thereon, the present invention can be used for the prevention or treatment of eye diseases, such as retinopathy of prematurity, corneal neovascularization, diabetic retinopathy, choroidal neovascular disease, macular degeneration (e.g., age-related macular degeneration), and the like.

In another aspect, the present invention is directed to a pharmaceutical composition for preventing or treating tumors including (a) a pharmaceutically effective amount of the antibody or fragment thereof according to the present invention, and (b) a pharmaceutically acceptable carrier. In another aspect, the present invention is directed to a method for preventing or treating tumors including administering the antibody or fragment thereof according to the present invention to a tumor patient. Furthermore, the present invention is directed to the use of the antibody or antigen-binding fragment thereof for inhibiting the mechanism of Ang2 and the use thereof for preventing or treating tumors based thereon.

Tumors, which are the diseases to which the composition can be applied, include typical tumors or cancers that overexpress Ang2, as well as tumors or cancers that do not express Ang2. Non-limiting examples of tumors or cancers that are targets of treatment include melanoma (*e*.*g*., metastatic malignant melanoma), kidney cancer (*e*.*g*., clear cell carcinoma), prostate cancer (*e*.*g*., hormone refractory prostate adenocarcinoma), pancreatic adenocarcinoma, breast cancer (in some cases, triple-negative breast cancer), colon cancer, lung cancer (*e*.*g*., non-small cell lung cancer), esophageal cancer, head and neck squamous cell carcinoma, liver cancer, ovarian cancer, cervical cancer, thyroid cancer, glioblastoma, glioma, leukemia, lymphoma, and other neoplastic carcinomas. In addition, the tumors or cancers according to the present invention include refractory or recurrent cancers that can be treated using the antibody of the present invention.

In another aspect, the present invention is directed to a composition for inhibiting angiogenesis including the antibody or fragment thereof as an active ingredient. In another aspect, the present invention is directed to a pharmaceutical composition for preventing and/or treating diseases associated with Ang2 activation and/or overproduction including the antibody or fragment thereof as an active ingredient.

The present invention provides, for example, a method for inhibiting angiogenesis including administering a therapeutically effective amount of the antibody or fragment thereof to a patient in need thereof. The method of inhibiting angiogenesis may further include identifying a patient in need of angiogenesis inhibition prior to the administration. In another aspect, the present invention is directed to a method for preventing and/or treating diseases associated with Ang2 activation and/or overproduction including administering a therapeutically effective amount of the antibody or fragment thereof to a patient in need thereof. The method for preventing and/or treating diseases associated with Ang2 activation and/or overproduction may further include identifying a patient in need of prevention and/or treatment of diseases associated with Ang2 activation and/or overproduction prior to the administration.

The pharmaceutical composition may further include a pharmaceutically acceptable carrier and may include, without being limited to, at least one selected from lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil and the like, which are commonly used for formulation of drugs. The pharmaceutical composition may further include at least one selected from the group consisting of a diluent, an excipient, a lubricant, a wetting agent, a sweetener, a flavoring agent, an emulsifier, a suspending agent, and a preservative which are commonly used in the preparation of pharmaceutical compositions.

The pharmaceutical composition or the antibody or antigen-binding fragment thereof may be orally or parenterally administered in an effective amount. The parenteral administration includes intravenous injection, subcutaneous injection, intramuscular injection, intraperitoneal injection, intradermal administration, topical administration, intranasal administration, intrapulmonary administration, intrarectal administration or the like. Upon oral administration, since proteins or peptides are digested, an oral composition should be coated with an active drug or formulated so as to protect the same from degradation in the stomach. In addition, the pharmaceutical composition may be administered using any device capable of delivering the active substance to target cells.

The content or dose of the antibody or fragment thereof in the pharmaceutical composition may vary depending on factors such as the formulation method, administration method, and age, body weight, gender, pathological conditions, diet, administration time, administration interval, administration route, excretion rate and responsiveness of the patient. For example, the daily dose of the antibody or fragment thereof may be within the range of 0.001 to 1,000 mg/kg, specifically 0.01 to 100 mg/kg, and more specifically 0.1 to 50 mg/kg, even more specifically 0.1 to 20 mg/kg, but the present invention is not limited thereto. The daily dose may be prepared by formulation into a single dosage form with a unit dose, formulation in an appropriate dose, or packaging in a multi-dose container.

The pharmaceutical composition may be administered in combination with other drugs such as other angiogenesis inhibitors or therapeutic agents for diseases associated with Ang2 activation and/or overproduction, and the dosage, administration method, and type of other drugs are appropriately selected according to the condition of the patient.

The pharmaceutical composition may be in the form of a solution, a suspension, a syrup or an emulsion in an oil or aqueous medium, or may be formulated in the form of an extract, a powder, a granule, a tablet or a capsule. The pharmaceutical composition may further include a dispersant or a stabilizer.

In particular, the pharmaceutical composition containing the antibody or fragment thereof may be formulated into an immunoliposome, since it includes the antibody and antigen-binding fragment thereof. A liposome containing an antibody can be prepared according to a method well-known in the art. The immunoliposome is a lipid composition containing phosphatidylcholine, cholesterol and polyethylene glycol-derivatized phosphatidylethanolamine, and can be prepared through reverse phase evaporation (KR Patent No. 10-2015-0089329). For example, the Fab' fragment of an antibody can be conjugated to a liposome via a disulfide exchange reaction.

Meanwhile, the antibody or fragment thereof specifically binds to Ang2 and thus can be used to determine whether or not Ang2 is activated and/or overproduced. Thus, in another aspect, the present invention is directed to a pharmaceutical composition for diagnosing Ang2 activation and/or overproduction and/or a disease associated with Ang2 activation and/or overproduction including the antibody or antigen-binding fragment thereof. In another aspect, the present invention is directed to a diagnostic method or providing information for diagnosis including treating a biological sample obtained from a patient with the antibody or fragment thereof, identifying the antigen-antibody reaction, and determining that the patient has a symptom of Ang2 activation and/or overproduction or a disease associated with Ang2 activation and/or overproduction when the antigen-antibody reaction is detected. The biological sample may be selected from the group consisting of cells, tissues, and bodily fluids derived from a patient.

The identifying whether or not the antigen-antibody reaction occurs can be carried out through various methods known in the art, for example, conventional enzyme reaction, fluorescence, luminescence and/or radiation detection, specifically, by a method selected from the group consisting of immunochromatography, immunohistochemistry, enzyme-linked immunosorbent assay (ELISA), radioimmunoassay (RIA), enzyme immunoassay (EIA), fluorescence immunoassay (FIA), luminescence immunoassay (LIA), Western blotting, and the like, but the present invention is not limited thereto.

The patient administered the pharmaceutical composition or the diagnosed patient may be a mammal including a primate including a human, a monkey or the like, or a rodent including a mouse, a rat or the like.

Diseases associated with the Ang2 activation and/or overproduction include: cancer; cancer metastasis; eye diseases such as retinopathy of prematurity, corneal neovascularization, diabetic retinopathy, choroidal neovascular disease, and macular degeneration (e.g., age-related macular degeneration); asthma; rheumatoid arthritis; psoriasis; inflammatory diseases such as pneumonia and chronic inflammation; and cardiovascular diseases such as hypertension or arteriosclerosis or sepsis. The cancer may overexpress Ang2 and may be solid cancer or hematological cancer, but is not limited thereto, may include at least one selected from the group consisting of squamous cell carcinoma, small cell lung cancer, non-small cell lung cancer, adenocarcinoma of the lung, squamous cell carcinoma of the lung, peritoneal cancer, skin cancer, skin or intraocular melanoma, rectal cancer, perianal cancer, esophageal cancer, small intestine cancer, endocrine gland cancer, parathyroid cancer, adrenal cancer, soft tissue sarcoma, urethral cancer, chronic or acute leukemia, lymphocytic lymphoma, hepatocellular carcinoma, gastrointestinal cancer, pancreatic cancer, glioblastoma, cervical cancer, ovarian cancer, liver cancer, bladder cancer, liver tumor, breast cancer (in some cases, triple-negative breast cancer), colon cancer, colorectal cancer, endometrial or uterine cancer, salivary gland cancer, kidney cancer, liver cancer, prostate cancer, vulvar cancer, thyroid cancer, liver cancer, head and neck cancer, brain cancer, osteosarcoma, and the like. The cancer may be primary cancer or metastatic cancer.

### Example

Hereinafter, the present invention will be described in more detail with reference to examples. However, it will be obvious to those skilled in the art that these examples are provided only for illustration of the present invention, and should not be construed as limiting the scope of the present invention.

The application of great amounts of anti-Ang2 antibodies by injection to eye diseases are expected to provide better effects. Since the volume suitable for injection into the eyes was limited, a stable formulation was required under higher concentration conditions than conventional substances. In order to prepare a more highly concentrated formulation than the conventional anti-Ang2 antibody, a high concentration formulation was successfully prepared using hot spot analysis based on the antibody structure and a formulation buffer.

### Example 1. Production of variants for improving physical properties

Efforts to improve the physical properties of the anti-Ang2 antibody clone O4 (including the heavy-chain variable region of SEQ ID NO: 34 and the light-chain variable region of SEQ ID NO: 32) were made.

**[Table 1]**

| Clone | Antibody | Sequence | SEQ. ID. No. |
|---|---|---|---|
| O4 | Heavy-chain variable region | CAGGTGCAGCTGGTAGAGTCTGGGGGAGGCCTG GTCAAGCCTGGGGGGTCCCTGAGACTCTCCTGT GCAGCCTCTGGATTCACCTTCAGTAGCTATAGC ATGAACTGGGTCCGCCAGGCTCCAGGGAAGGGG CTGGAGTGGGTCTCATCCATTAGTGCTAGTGAT GGTGCCACATACTACGCAGACTCCGTGAGGGGC CGGTTCACCATCTCCAGAGACAATTCCAGGAGC ACACTGTATCTGCAAATGAACAGTCTGAGAGCC GAGGACACGGCCGTGTATTACTGTGCGAAAACT CTCGCGGGATATAGTGGCCCAATGGGTGGCATG GACGTCTGGGGCCAAGGGACCACGGTCACCGTC TCCTCA | 40 |
| | | QVQLVESGGGLVKPGGSLRLSCAASGFTFSSYS MNWVRQAPGKGLEWVSSISASDGATYYADSVRG RFTISRDNSRSTLYLQMNSLRAEDTAVYYCAKT LAGYSGPMGGMDVWGQGTTVTVSS | 34 |
| | Light-chain variable region | GACATCCAGATGACCCAGTCTCCATCCTCACTG TCTGCATCTGTAGGAGACAGAGTCACCATCACT TGTCGGGCGAGTCGGGACATTAGCAACTATTTA GCCTGGTATCAGCAGAAACCAGGGAAAGCCCCT AAGTCCCTGATCTATGGAGCATCCAATTTACAA AGTGGGGTCTCATCACAGTTCAGCGGCAGTGGA TCCGGGACAGATTTCACCCTCACCATCAACAGC CTGCAGCCTGAAGATTCTGCAACTTATTACTGT CAACAGTACTATAGTTACCCGCTCACTTTTGGC GGAGGGACCAAGGTGGATATCAAACGT | 38 |
| | | DIQMTQSPSSLSASVGDRVTITCRASRDISNYL AWYQQKPGKAPKSLIYGASNLQSGVSSQFSGSG SGTDFTLTINSLQPEDSATYYCQQYYSYPLTFG GGTKVDIKR | 32 |

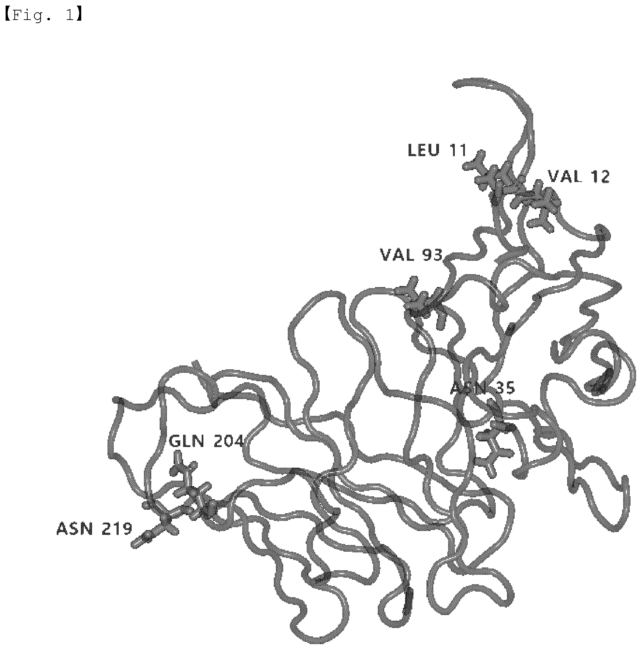

Amino acids that are likely to cause aggregation of the anti-Ang2 antibody were analyzed using Discovery Studio (BIOBIA) software. The amino acids Leu 11, Val 12, Asn 35, Val 93 in VH, and Gln 61 and Asn 76 in VL were analyzed as hot spots that affect physical properties (FIG. 1, Table 2). Amino acids analyzed as hot spots were mutated, expressed in variants with improved physical properties and selected.

**[Table 2]**

| Antibody | Variation | AHO No. |
|---|---|---|
| Heavy chain | L11T | L11T |
| | V12S | V13S |
| | N35T | N42T |
| | V93S | V103S |
| Light chain | Q61R | Q77R |
| | N76S | N94S |

### Example 2. Production of variants for improving physical properties

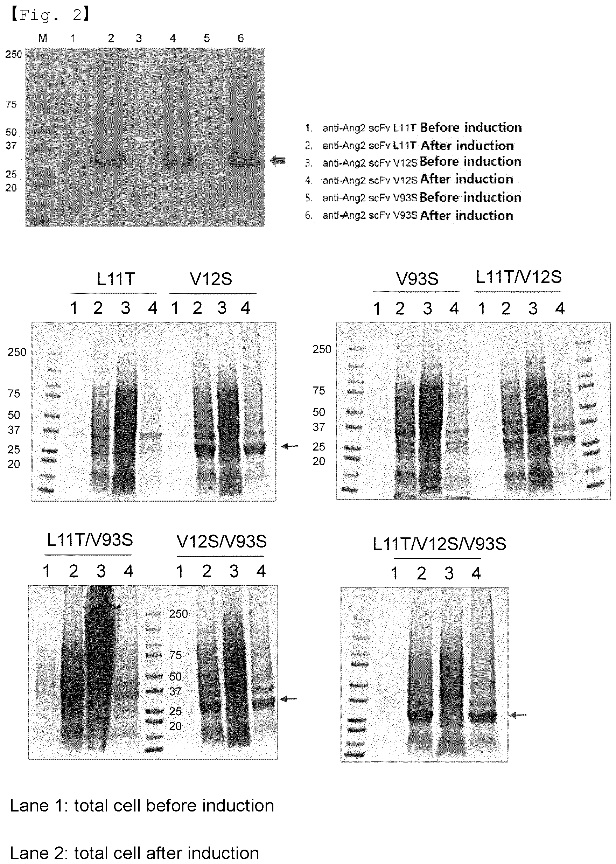

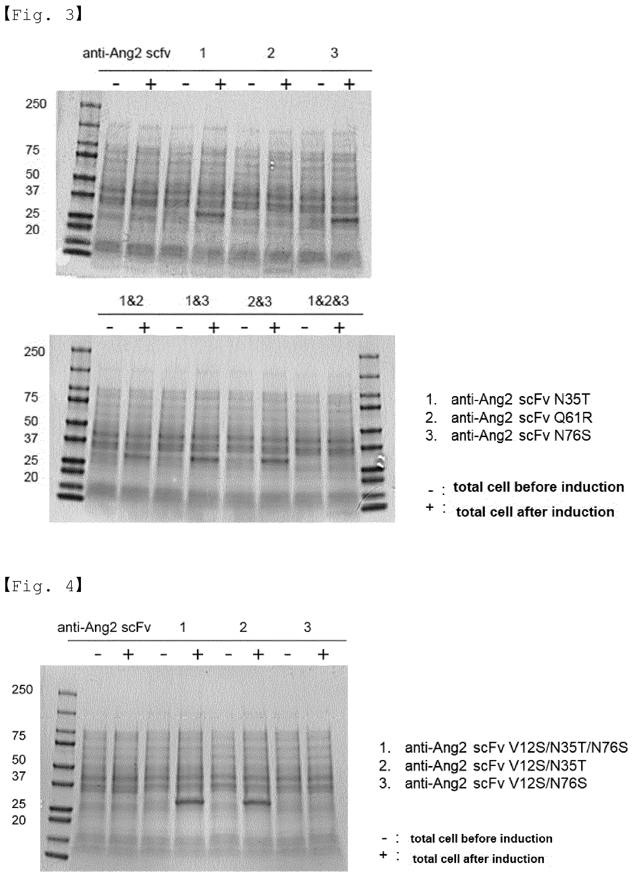

In order to improve the physical properties of the anti-Ang2 antibody (O4), amino acids selected through hot spot analysis were substituted. The expression test of the amino acid-changed construct was performed. Clones with point mutations in the anti-Ang2 antibody (O4) gene were amplified using PCR. Cloning was completed by inserting the amplified gene into an expression vector (Merck Millipore, pET 22b(+)). Transformation was performed by inserting the cloned plasmid into an expression host cell (Merck Millipore, BL21(DE3)). For the expression test, 100 µg/ml ampicillin was added to 2XYT medium, and the transformed cells were inoculated and cultured at 37°C for 8 hours. After lowering the temperature to 25°C, 0.5 mM IPTG was added to induce expression and incubation was performed for 18 hours. Cells were harvested after centrifugation at 8,000 rpm for 10 minutes. The precipitated cells were suspended in 50 mM Tris, 150 mM NaCl pH 7.4 buffer and disrupted by ultrasonication. The disrupted cells were separated from the supernatant by centrifugation at 1,3000 rpm for 1 hour and expression was detected by SDS-PAGE (FIGS. 2, 3 and 4).

### Example 3. ScFv production of optimized anti-Ang2 antibody

The anti-Ang2 antibody with improved physical properties was cloned into a pET-22b vector (Novagen) in order to express the antibody in *E. coli.* Colonies generated by transformation into BL21 (DE3) were selected and 1% *E. coli* pre-cultured at 37°C at 200 rpm in LB medium containing 100 µg/ml of ampicillin was inoculated in LB (lysogeny broth) medium containing 100 µg/ml of ampicillin. The colonies were cultured at 37°C and 200 rpm, the temperature of the incubator was lowered to 20°C when OD₆₀₀ reached 0.6 to 0.8 and, and 0.5 mM IPTG was added thereto, followed by culturing for 16 hours.

The cultured *E. coli* was collected by centrifugation at 8,000 rpm for 10 minutes. After removing the medium, the cells were resuspended using 10 ml lysis buffer (50 mM Tris-HCl pH 7.4, 150 mM NaCl, 1 mM PMSF (phenylmethylsulfonyl fluoride) per g of cell weight. The cells were disrupted using a sonicator under conditions (power: 20W, rest: 3 sec, Work: 3 sec, Time: 10 min). The disrupted cells were centrifuged at 13,000 rpm for 1 hour to separate the supernatant and the precipitate from each other.

ScFv was expressed in an insoluble form and subjected to pellet washing to perform refolding. After homogenization with a homogenizer using 50 mM Tris-HCl pH 7.4 and 150 mM NaCl buffer, the pellet was washed twice by centrifugation at 13,000 rpm for 1 hour. *E. coli-*derived materials remaining in the pellet were removed using buffer containing 50 mM Tris-HCl pH 7.4, 150 mM NaCl, 2 M urea, and 0.5% Triton X-100, and were repeatedly washed three times with pH 7.4 buffer containing 50 mM Tris-HCl and 150 mM NaCl. After resuspension of the inclusion body with buffer containing 50 mM Tris-HCl pH 7.4, 150 mM NaCl, 8 M urea, and 10 mM DTT, reaction was performed for about 30 minutes to obtain the unfolded ScFv, and centrifuged at 13,000 rpm for 1 hour to separate the supernatant from the precipitate.

The ScFv antibody was refolded by removing urea through step dialysis. The dialysis buffer was prepared by lowering the concentration of urea by 1/2 based on 50 mM Tris-HCl pH 7.4 and 150 mM NaCl. In the 4-2-1 M urea concentration range where most structures are formed, 0.1 M L-arginine and 10% glycerol were added to inhibit aggregation and thereby perform refolding. The refolded scFv antibody was separated and purified using HisTrap and Capo L columns.

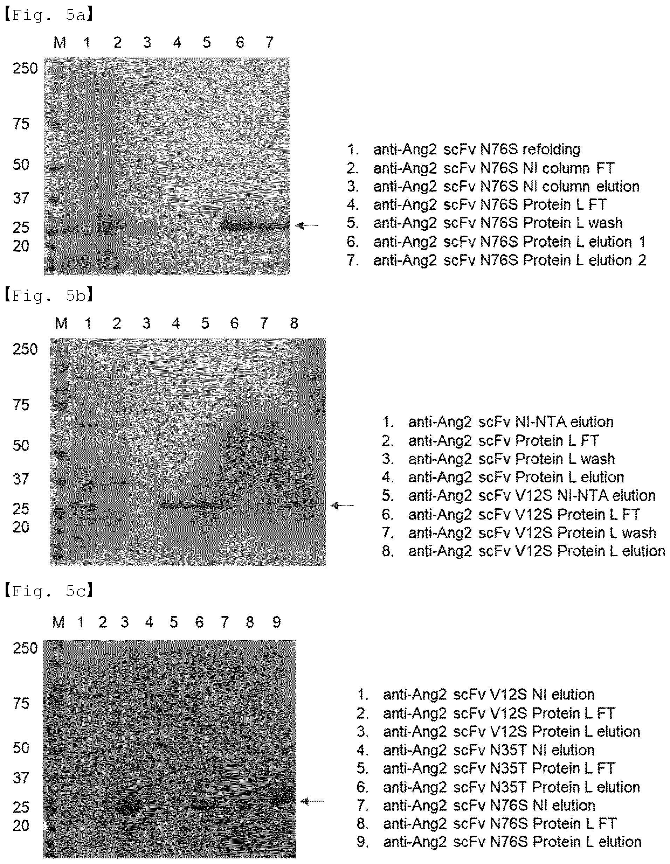

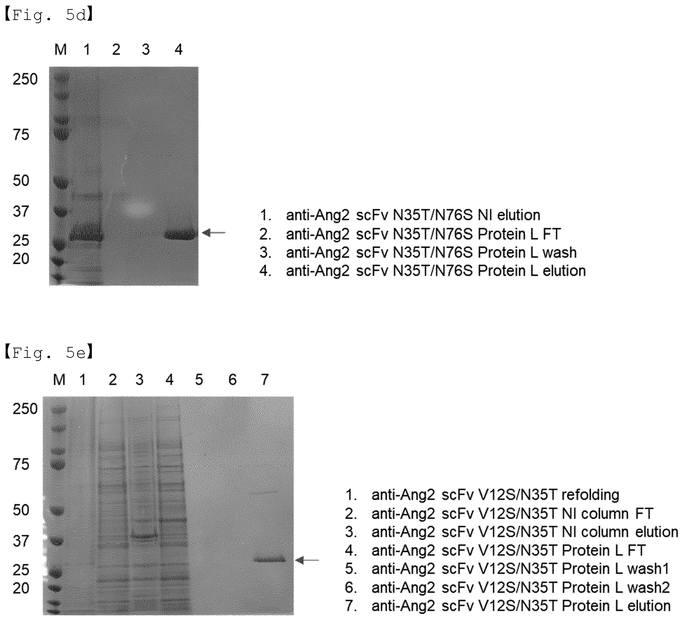

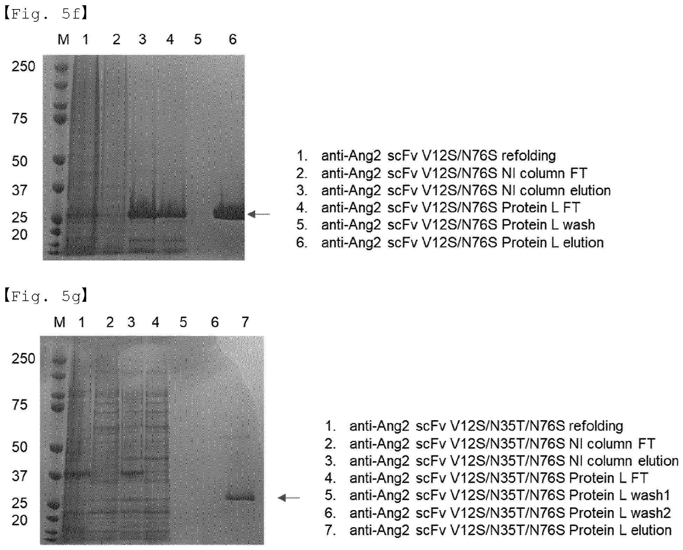

Separation and purification were performed in the following process. Purification was performed using an AKTA purifier (GE healthcare) system and a 5 ml HisTrap packing column was used. After equilibration with pH 7.4 buffer containing 50 mM Tris, 500 mM NaCl, and 10 mM imidazole in a column volume corresponding to 10 times the HisTrap column volume, the ScFv antibody sample was allowed to flow through the HisTrap column at a flow rate of 3 ml/min to bind to the resin in the column. In order to remove non-specific binding substances present in the resin, pH 7.4 buffer containing 50 mM Tris, 150 mM NaCl, and 400 mM imidazole in an amount of corresponding to about 10 times the column volume was allowed to flow and then pH 7.4 buffer containing 50 mM Tris, 150 mM NaCl, and 400 mM imidazole was allowed to flow at the concentration gradient to elute the scFv antibody. The eluted antibody sample was purified using a 5 ml Capto L column as follows. After equilibration of the Capto L column with pH 7.4 buffer containing 50 mM Tris and 150 mM NaCl, the sample was allowed to flow through the Capto L column at a flow rate of 3 ml/min and bind to the resin in the column. PBS buffer was allowed to pass through the column in an amount of about 10 times the column volume to remove non-specifically bound substances. Non-specifically bound substances that could not be removed with the PBS buffer were removed by flowing 10 CV of pH 5.5 buffer containing 50 mM MES, 400 mM NaCl, and 0.2% tween 20. pH 2.5 buffer containing 0.1 M glycine and 50 mM NaCl was allowed to flow through the column in an amount of about 10 times the column volume to elute the scFv antibody. The pH of the elution sample was neutralized with pH 7.4 buffer of 1 M Tris. FIGS. 5A to 5G show the results of SDS-PAGE on the protein obtained after final purification. As a result, a highly pure ScFv antibody was obtained.

The specific sequence of the anti-Ang2 antibody with improved physical properties is as follows.

**[Table 3]**

| Variation | Antibody | Sequence | No. |
|---|---|---|---|
| V12S | VH | 1 QVQLVESGGG LSKPGGSLRL SCAASGFTFS SYSMNWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 31 |
| | VL | 1 DIQMTQSPSS LSASVGDRVT ITCRASRDIS NYLAWYQQKP 41 GKAPKSLIYG ASNLQSGVSS QFSGSGSGTD FTLTINSLQP 81 EDSATYYCQQ YYSYPLTFGG GTKVDIKR | 32 |
| N35T | VH | 1 QVQLVESGGG LVKPGGSLRL SCAASGFTFS SYSMTWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 33 |
| | VL | 1 DIQMTQSPSS LSASVGDRVT ITCRASRDIS NYLAWYQQKP 41 GKAPKSLIYG ASNLQSGVSS QFSGSGSGTD FTLTINSLQP 81 EDSATYYCQQ YYSYPLTFGG GTKVDIKR | 32 |
| N76S | VH | 1 QVQLVESGGG LVKPGGSLRL SCAASGFTFS SYSMNWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 34 |
| | VL | 1 DIQMTQSPSS LSASVGDRVT ITCRASRDIS NYLAWYQQKP 41 GKAPKSLIYG ASNLQSGVSS QFSGSGSGTD FTLTISSLQP 81 EDSATYYCQQ YYSYPLTFGG GTKVDIKR | 35 |
| V12S/N35T | VH | 1 QVQLVESGGG LSKPGGSLRL SCAASGFTFS SYSMTWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 36 |
| | VL | 1 DIQMTQSPSS LSASVGDRVT ITCRASRDIS NYLAWYQQKP 41 GKAPKSLIYG ASNLQSGVSS QFSGSGSGTD FTLTINSLQP 81 EDSATYYCQQ YYSYPLTFGG GTKVDIKR | 32 |
| V12S/N76S | VH | 1 QVQLVESGGG LSKPGGSLRL SCAASGFTFS SYSMNWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 31 |
| | VL | 1 DIQMTQSPSS LSASVGDRVT ITCRASRDIS NYLAWYQQKP 41 GKAPKSLIYG ASNLQSGVSS QFSGSGSGTD FTLTISSLQP 81 EDSATYYCQQ YYSYPLTFGG GTKVDIKR | 35 |
| N35T/N76S | VH | 1 QVQLVESGGG LVKPGGSLRL SCAASGFTFS SYSMTWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 33 |
| | VL | 1 DIQMTQSPSS LSASVGDRVT ITCRASRDIS NYLAWYQQKP 41 GKAPKSLIYG ASNLQSGVSS QFSGSGSGTD FTLTISSLQP 81 EDSATYYCQQ YYSYPLTFGG GTKVDIKR | 35 |
| V12S/N35T/N76S | VH | 1 QVQLVESGGG LSKPGGSLRL SCAASGFTFS SYSMTWVRQA 41 PGKGLEWVSS ISASDGATYY ADSVRGRFTI SRDNSRSTLY 81 LQMNSLRAED TAVYYCAKTL AGYSGPMGGM DVWGQGTTVT 121 VSS | 36 |
| | VL | 1 DIQMTQSPSS LSASVGDRVT ITCRASRDIS NYLAWYQQKP 41 GKAPKSLIYG ASNLQSGVSS QFSGSGSGTD FTLTISSLQP 81 EDSATYYCQQ YYSYPLTFGG GTKVDIKR | 35 |

### Example 4. ScFv concentration of optimized anti-Ang2 antibody

In order to analyze the highest concentration that does not cause aggregation, concentration was performed using centrifugation. The purified anti-Ang2 antibody was dialyzed with PBS and a formulation buffer (10 mM sodium phosphate, 40 mM NaCl, 0.03% polysorbate, 5% sucrose, pH 6.2) containing the formulation composition used in commercial products to perform buffer exchange. 10 ml of PBS or formulation buffer was added to a Viva Spin Turbo 5,000 MWCO PEG tube (Sartorius) and centrifuged at 3,400 g for 10 minutes to equilibrate and 10 ml of the buffer-exchanged sample was injected into a tube and concentrated by centrifugation at 3,400 g and at 4°C for 10 minutes. The absorbance was measured with NanoDrop (Thermo) to measure concentration (Table 4).

**[Table 4]**

| Sample | ScFv PBS | ScFv formulation buffer | V12S PBS | V12S formulation buffer |
|---|---|---|---|---|
| Maximum concentration | 43.4 mg/ml | 57.25 mg/ml | 92.1 mg/ml | 137.97 mg/ml |
| Sample | N35T | N35T | N76S | N76S |
| | PBS | formulation buffer | PBS | formulation buffer |
| Maximum concentration | 55.88 mg/ml | 87.12 | 107.35 mg/ml | 167.32 mg/ml |
| Sample | V12S/N35T PBS | V12S/N76S PBS | N35T/N76S PBS | V12S/N35T/N76S PBS |
| Maximum concentration | 95.64 mg/ml | 95.99 | 96.31 mg/ml | 99.86 mg/ml |

### Example 5. Analysis of binding specificity of optimized anti-Ang2 ScFv antibody

For binding specificity analysis, binding constants were measured using the Octet system (Pall Fortebio LLC. US).

The binding ability of the purified ScFv antibody to human Ang2 and mouse Ang2 was measured using the Octet system (Fortebio Inc. US). The AR2G biosensor was hydrated for 20 minutes. The AR2G biosensor was activated using a solution of 5% s-NHS and 5% EDC in 90% triple distilled water. The anti-Ang2 antibody was immobilized on the AR2G sensor at a concentration of 1 ug/ml. The antibody was quenched with 1 M ethanolamine. The antibody was equilibrated with PBS, and the association kinetics were measured at concentrations of 50, 40, 30, and 20 nM of human Ang2 to calculate the association rate constant (kₐ), dissociation rate constant (k_{dis}), and association constant (K_{D}) (Table 5).

**[Table 5]**

| Sample | kₐ (1/Ms) | K_{dis} (1/s) | K_{D} (M) |
|---|---|---|---|
| ScFv N76S (hAng2) | 5.49E+05 | 1.72E-04 | 1.13E-10 |
| ScFv N76S (mAng2) | 7.33E+05 | 1.69E-04 | 2.31E-10 |

### Example 6: Analysis of thermal stability of anti-Ang2 scFv antibody

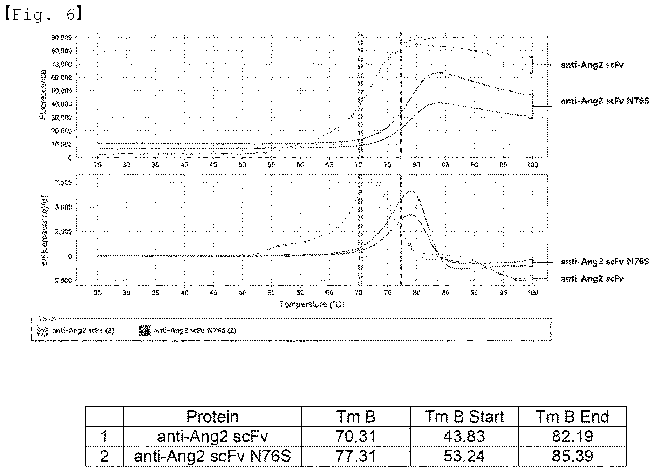

To analyze the thermal stability of the anti-Ang2 scFv antibody, the Tm was measured using RT-PCR. The analysis sample was prepared to have a composition of 5 µl of protein thermal shift buffer (Thermo Fisher), 12.5 µl of anti-Ang2 scFv antibody (1 mg/ml in PBS pH 7.4 buffer), and 2.5 µl of 8X protein thermal shift dye and to enable two repetitions of the experiment. Tm analysis was performed by increasing the temperature from 25°C to 99°C at a rate of 1.6 °C/s using the melt curve experiment method in the QuantStudio 5 thermocycler (Thermo Fisher) using the melt curve experiment method in the QuantStudio 5 thermocycler (Thermo Fisher), measuring fluorescence intensity (absorbance a (580±10 nm)-absorbance b (623±14 nm) and calculating Tm values using protein thermal shift software (Thermo Fisher). The Tm B value of the anti-Ang2 scFv antibody calculated in accordance with the Boltzmann equation was 77.31°C, which is an increase of more than 7°C compared to before the technical modification, indicating that the thermal stability was greatly improved due to the technical modification (FIG. 6).

### Example 7: Evaluation of anti-angiogenic efficacy of anti-Ang2 scFv antibody using mouse CNV model

In order to determine whether or not the anti-Ang2 scFv antibody has an angiogenesis inhibitory effect, a drug efficacy test was conducted in a laser-induced choroidal neovascularization mouse model. The efficacy was tested with a commercial drug, aflibercept, as a control. After general anesthesia of the mice with ketamine, anesthetic eye drops were applied to the eyes for additional local anesthesia, and a mydriatic agent was further added to the eyes to induce mydriasis. The mouse was put on the sacrificial table and a laser burn was induced using the Micron-IV under CNV induction conditions (wavelength 532 nm, diameter 50 um, duration 80 mS, power level 200 mW) to destroy Bruch's membrane. Lesions in which bubbling was not observed during the laser burn induction process were classified as unsuccessful laser burns and were excluded from the analysis and statistical processing process based on the exclusion criteria modified from the criteria proposed by Gong Y. et al.

In order to determine the effect of inhibiting angiogenesis by CNV induction and the drug, mice were anesthetized with ketamine on day 10 after induction of CNV, and a fluorescent contrast agent was intraperitoneally injected into the mice. Anesthetic eye drops were added dropwise to the eyes to induce additional local anesthesia and a mydriatic agent was further added thereto to induce mydriasis. The mouse was placed on the sacrifice table, a Micron-IV's imaging camera was used to focus on the fundus, a lubricating gel was applied to the eyeball, and the OCT lens was brought into contact with the cornea. After FFA/OCT imaging, a drop of antibiotic ophthalmic solution was added to the eye of the mouse. Analysis of FFA and OCT images was performed using the 'Image-J' program.

An electroretinogram (ERG) test was performed to determine the optic nerve recovery effect. The mouse was dark-adapted in a dark room 12 hours before the ERG evaluation. On the day of evaluation (11 days after CNV induction), after general anesthesia with Rumpun^{®} and Ketamine^{®}, Alkyne^{®} was added to the eye to induce additional local anesthesia and a mydriatic agent was further added thereto to induce mydriasis. The mouse was placed on an ERG sacrificial table, and ERG probes were brought into contact with the tail, head, and cornea, respectively. ERG was measured as an electroretinogram change for a single flash stimulus (0.9 log cds/m² (10 responses/intensity)). When the ERG evaluation was completed, a drop of Tobrex was added to the eye of the mouse. ERG analysis was performed using the 'LabScribeERG (iWorx DataAcquisition Software)' program.

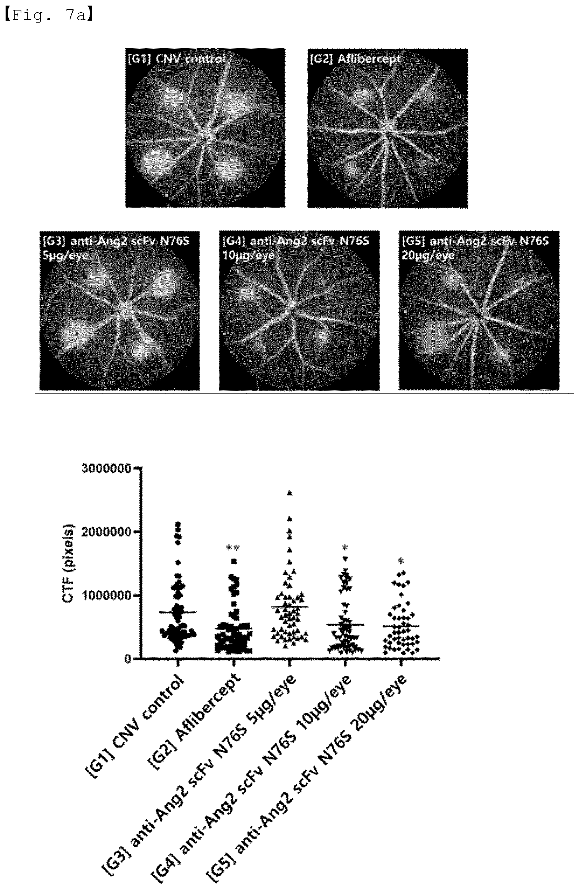

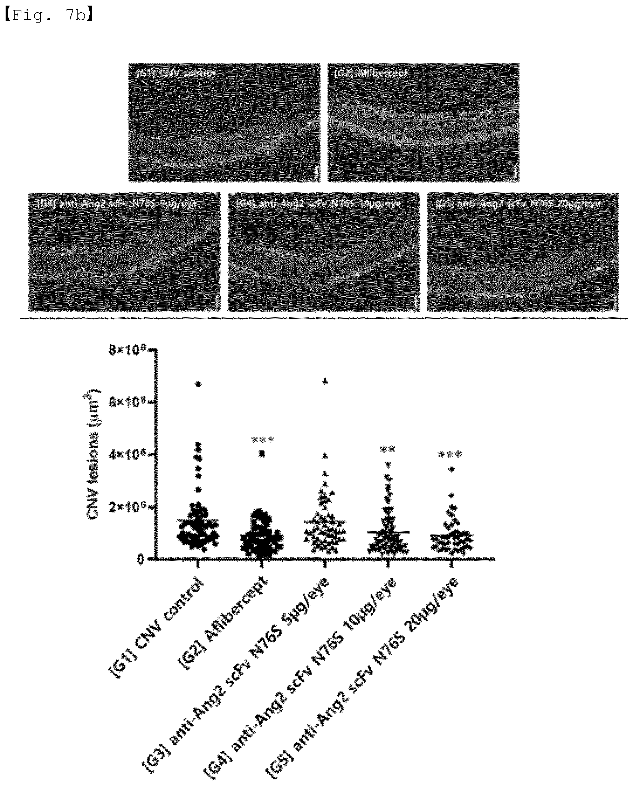

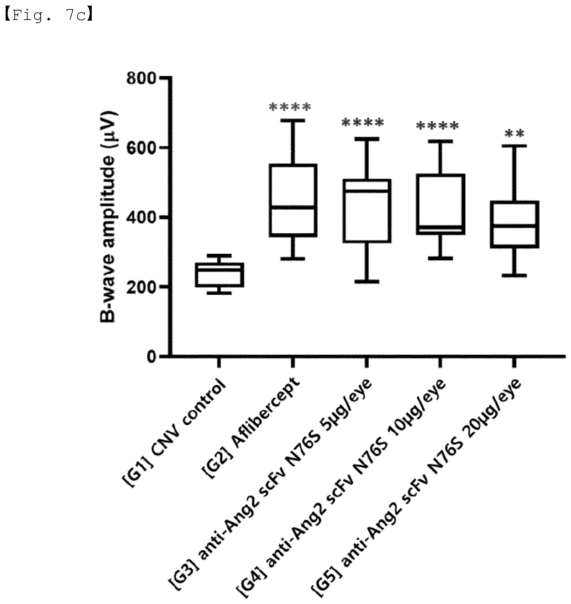

Aflibercept was diluted at a dose of 20 µg/µl/eye in sterile PBS and the dilution was administered once through IVT (intravitreal injection) to the anti-Ang2 scFv antibody at doses of 5 pg/pl/eye, 10 µg/µl/eye and 20 µg/µl/eye on the day after CNV induction. On the 10^{th} day after CNV induction, retinal image evaluation was performed using FFA (fundus fluorescein angiography) and OCT (optical coherence tomography), and the sizes and cross-sectional areas of CNV lesions were measured based thereon, followed by comparison and evaluation. In addition, the changes in electroretinogram for mono-flash (0.9 log cds/m²) stimulation in the scotopic ERG (electroretinogram) on the 11^{th} day after CNV induction were summarized as the amplitude from A-wave to B-wave, followed by comparison and evaluation. As a result, in terms of the size of the CNV lesions measured on FFA, the lesion sizes of the aflibercept-administered group *(P<0.01)* and the anti-Ang2 scFv antibody-administered group at 10 µg/µl/eye and 20 µg/µl/eye was statistically significantly reduced than the lesions in the vehicle-administered group (*P<0.05* for each) (FIG. 7A). In addition, the results of measuring the volume of CNV lesions measured on OCT showed that the volumes of CNV lesions in the groups administered 10 µg/µl/eye and 20 µg/µl/eye of anti-Ang2 scFv antibody were statistically significantly reduced (respectively *P<0.01* and *P<0.001*) (FIG. 7B). When the electroretinogram on the ERG was compared with the vehicle-administered group, both the aflibercept-administered group and the anti-Ang2 scFv antibody-administered group (G3, G4, and G5) exhibited significantly increased electroretinogram compared to the CNV control group, G1 group (*P<0.0001, P<0.0001, P<0.0001* and *P<0.01,* respectively) (FIG. 7C).

### [Industrial applicability]

The antibody or fragment thereof according to the present invention includes mutations in the framework region, thereby improving the solubility, enabling concentration at a high level, and thus improving antibody productivity.

Although specific configurations of the present invention have been described in detail, those skilled in the art will appreciate that this detailed description is provided as preferred embodiments and should not be construed as limiting the scope of the present invention. Therefore, the substantial scope of the present invention is defined by the accompanying filed claims and equivalents thereto.

### [Sequence Listing Free Text]

An electronic file is attached.

## Claims

1. A modified antibody and a fragment thereof in which at least one amino acid selected from the group consisting of amino acids at positions 13 and 42 of a heavy-chain variable region (based on AHO numbering system) and an amino acid at position 94 of a light-chain variable region (based on AHO numbering system) is substituted.

2. The modified antibody and fragment thereof according to claim 1, wherein the amino acids at position 13 and position 42 (based on AHO numbering system) in the heavy-chain variable region and the amino acid at position 94 (based on AHO numbering system) in the light-chain variable region are substituted with serine (S) or threonine (T).

3. The modified antibody and fragment thereof according to claim 1, wherein the substitutions of amino acids comprise substitutions of amino acids at one or more positions (based on AHO numbering system) selected from the group consisting of:
substitution of valine (V) at position 13 in the heavy-chain variable region with serine (S);
substitution of asparagine (N) at position 42 in the heavy-chain variable region with threonine (T); and
substitution of asparagine (N) at position 94 in the light-chain variable region with serine (S).

4. The modified antibody and fragment thereof according to claim 1, wherein the heavy-chain variable region having amino acid substitution comprises a framework region having a sequence of QVQLVESGGGLX₁KPGGSLRLSCAAS (wherein X₁ is S) and/or MX₂WVRQAPGKGLEWVSS (wherein X₂ is T) .

5. The modified antibody and fragment thereof according to claim 1, wherein the light-chain variable region having amino acid substitution comprises a framework region having a sequence of NLQSGVSSQFSGSGSGTDFTLTIX₃SLQPEDSATYYC (wherein X₃ is S) .

6. The modified antibody and fragment thereof according to claim 1, wherein the modified antibody or fragment thereof comprises one or more framework regions selected from the group consisting of:
QVQLVESGGGLX₁KPGGSLRLSCAAS (in which X₁ is S);
MX₂WVRQAPGKGLEWVSS (in which X₂ is T); and
NLQSGVSSQFSGSGSGTDFTLTIX₃SLQPEDSATYYC (in which X₃ is S) .

7. The modified antibody and fragment thereof according to claim 1, wherein the antibody or fragment thereof comprises:
a heavy-chain variable region including a heavy-chain CDR1 selected from the group consisting of SEQ ID NOS: 1, 7, 13, 19 and 25;
a heavy-chain CDR2 selected from the group consisting of SEQ ID NOS: 2, 8, 14, 20 and 26; and
a heavy-chain CDR3 selected from the group consisting of SEQ ID NOS: 3, 9, 15, 21, 27, 43, 44 and 45; and
a light-chain variable region including a light-chain CDR1 selected from the group consisting of SEQ ID NOS: 4, 10, 16, 22, and 28;
a light-chain CDR2 selected from the group consisting of SEQ ID NOS: 5, 11, 17, 23 and 29; and
a light-chain CDR3 selected from the group consisting of SEQ ID NOS: 6, 12, 18, 24 and 30.

8. The modified antibody and fragment thereof according to claim 1, wherein the modified antibody and fragment thereof comprises a heavy-chain variable region selected from the group consisting of SEQ ID NOS: 31, 33 and 36.

9. The modified antibody and fragment thereof according to claim 1, wherein the modified antibody and fragment thereof comprises a light-chain variable region of SEQ ID NO: 35.

10. The modified antibody and fragment thereof according to claim 1, wherein the modified antibody and fragment thereof comprises:
a heavy-chain variable region of SEQ ID NO: 31 and a light-chain variable region of SEQ ID NO: 32;
a heavy-chain variable region of SEQ ID NO: 33 and a light-chain variable region of SEQ ID NO: 32;
a heavy-chain variable region of SEQ ID NO: 34 and a light-chain variable region of SEQ ID NO: 35;
a heavy-chain variable region of SEQ ID NO: 36 and a light-chain variable region of SEQ ID NO: 32;
a heavy-chain variable region of SEQ ID NO: 31 and a light-chain variable region of SEQ ID NO: 35;
a heavy-chain variable region of SEQ ID NO: 33 and a light-chain variable region of SEQ ID NO: 35; or
a heavy-chain variable region of SEQ ID NO: 36 and a light-chain variable region of SEQ ID NO: 35.

11. A method of producing a modified antibody and a fragment thereof in which at least one amino acid selected from the group consisting of amino acids at positions 13 and 42 of a heavy-chain variable region (based on AHO numbering system) and amino acid at position 94 of a light-chain variable region (based on AHO numbering system) is substituted.

12. The method according to claim 11, wherein the amino acids at position 13 and position 42 (based on AHO numbering system) in the heavy-chain variable region and the amino acid at position 94 (based on AHO numbering system) in the light-chain variable region are substituted with serine (S) or threonine (T).

13. The method according to claim 11, wherein the substitutions of amino acids comprise substitutions of amino acids at one or more positions (based on AHO numbering system) selected from the group consisting of:
substitution of valine (V) at position 13 in the heavy-chain variable region with serine (S);
substitution of asparagine (N) at position 42 in the heavy-chain variable region with threonine (T); and
substitution of asparagine (N) at position 94 in the light-chain variable region with serine (S).

14. A nucleic acid encoding the modified antibody or fragment thereof according to any one of claims 1 to 10.

15. An expression vector comprising the nucleic acid according to claim 14.

16. A cell transformed with the expression vector according to claim 15.

17. A method for producing a modified antibody or fragment thereof, the method comprising:
(a) culturing the cell according to claim 16; and
(b) collecting an antibody or antigen-binding
